(19) Europäisches Patentamt
European Patent Office
Office européen des brevets

(11) Veröffentlichungsnummer: **0 430 033 A2**

# (12) EUROPÄISCHE PATENTANMELDUNG

(21) Anmeldenummer: **90122174.7**

(22) Anmeldetag: **20.11.90**

(51) Int. Cl.5: **C07C 259/10**, A01N 37/28, C07C 243/38, C07D 295/32, A01N 43/84, C07C 251/84, C07C 251/76, A01N 43/40, C07C 251/86

(30) Priorität: **24.11.89 CH 4210/89**

(43) Veröffentlichungstag der Anmeldung: **05.06.91 Patentblatt 91/23**

(84) Benannte Vertragsstaaten: **AT BE CH DE DK ES FR GB GR IT LI LU NL SE Patentblatt**

(71) Anmelder: **CIBA-GEIGY AG**
**Klybeckstrasse 141**
**CH-4002 Basel(CH)**

(72) Erfinder: **Riebli, Peter**
**Bienenheim**
**CH-6074 Giswil(CH)**

(74) Vertreter: **Zumstein, Fritz, Dr. et al**
**Zumstein & Klingseisen Patentanwälte**
**Bräuhausstrasse 4**
**W-8000 München 2(DE)**

## (54) Mikrobizide Mittel.

(57) Neue 1-Aryl-2-naphthoesäureamide der Formel

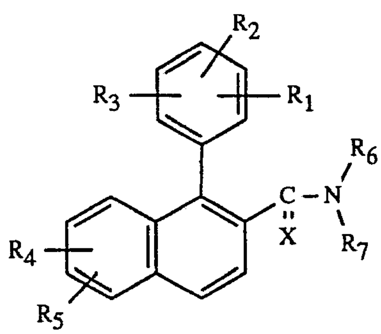

worin bedeuten:
$R_1$, $R_2$, $R_3$, $R_4$ und $R_5$ unabhängig voneinander Wasserstoff, Halogen, Nitro, Cyano, unsubstituiertes oder durch Halogen und/oder $C_1$-$C_3$-Alkoxy ein- oder mehrfach substituiertes $C_1$-$C_6$-Alkyl, oder $C_3$-$C_6$-Cycloalkyl, $OR_8$, N-$(R_9)R_{10}$, $C(O)OR_9$, $CON(R_9)R_{10}$ oder $N(R_9)COR_{11}$, oder worin zwei benachbarte Positionen an den aromatischen Ringen durch eine unsubstituierte oder durch Fluor ein- oder mehrfach substituierte Methylendioxy- oder Aethylendioxy-Gruppe überbrückt sind,
$R_8$ Wasserstoff oder $C_1$-$C_6$Alkyl, das unsubstituiert oder durch $C_1$-$C_3$-Alkoxy oder ein-oder mehrfach durch Halogen substituiert ist, oder $C_3$-$C_4$-Alkenyl, Propargyl, 3-Halogen-2-propinyl,

EP 0 430 033 A2

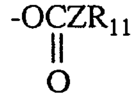 oder $COR_{11}$;

$R_9$ und $R_{10}$ unabhängig voneinander Wasserstoff oder $C_1$-$C_4$-Alkyl;
$R_{11}$ $C_1$-$C_4$-Alkyl;
Z Sauerstoff oder NH;
$R_6$ Wasserstoff, unsubstituiertes oder durch $C_1$-$C_4$-Alkoxy, Halogen und/oder Cyano ein-oder mehrfach substituiertes $C_1$-$C_6$-Alkyl, oder $C_3$-$C_6$-Cycloalkyl, $C_3$-$C_7$-Alkenyl, $C_3$-$C_7$-Alkinyl oder durch Halogen substituiertes $C_3$-$C_7$-Alkinyl oder

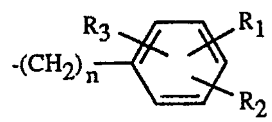 ;

n 0 oder 1;
$R_7$ X-$R_{12}$,

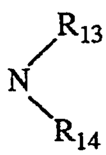 oder 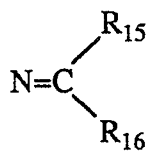 ;

X Sauerstoff oder Schwefel;
$R_{12}$ die Definitionen von $R_6$ oder

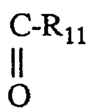 ;

$R_{13}$ und $R_{14}$ unabhängig voneinander die Definitionen von $R_{12}$, oder $R_{13}$ und $R_{14}$ gemeinsam eine $C_4$-$C_7$-Alkylenkette bilden, welche mit dem sie verbindenden Stickstoffatom einen unsubstituierten oder durch $C_1$-$C_4$-Alkyl mono- oder disubstituierten Heterocyclus bilden kann, wobei eine der Methylengruppen aus dieser Kette durch O, S oder $NR_9$ ersetzt sein kann;
$R_{15}$ und $R_{16}$ unabhängig voneinander die Definitionen von $R_6$, oder $R_{15}$ und $R_{16}$ gemeinsam eine $C_4$-$C_7$-Alkylenkette bilden, wobei diese durch $C_1$-$C_4$-Alkyl mono- oder disubstituiert sein kann und/oder eine der Methylengruppe aus dieser Kette durch O, S oder $NR_9$ ersetzt sein kann;
einschliesslich der Säureadditionsalze der Verbindungen der Formel I.

Die neuen Wirkstoffe besitzen pflanzenschützende Eigenschaften und eignen sich insbesondere zum Schutz von Pflanzen gegen den Befall von phytopathogenen Mikroorganismen wie Fungi, Bakterien und Viren.

## MIKROBIZIDE MITTEL

Die vorliegende Erfindung betrifft neue substituierte 1-Aryl-2-naphthoesäureamide, deren Herstellung, sowie mikrobizide Mittel, die als Wirkstoff mindestens eine dieser Verbindungen enthalten. Die Erfindung betrifft ferner die Herstellung der genannten Mittel und die Verwendung der neuen Wirkstoffe und Mittel zur Bekämpfung schädlicher Mikroorganismen, insbesondere pflanzenschädigender Pilze.

Die erfindungsgemässen Verbindungen entsprechen der allgemeinen Formel I

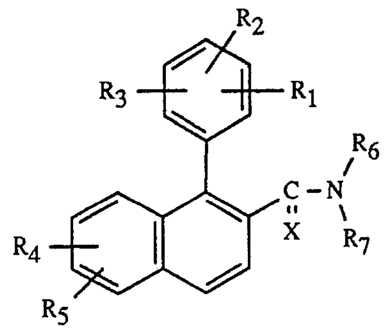

(I)

worin bedeuten:

$R_1$, $R_2$, $R_3$, $R_4$ und $R_5$ unabhängig voneinander Wasserstoff, Halogen, Nitro, Cyano, unsubstituiertes oder durch Halogen und/oder $C_1$-$C_3$-Alkoxy ein- oder mehrfach substituiertes $C_1$-$C_6$-Alkyl, oder $C_3$-$C_6$-Cycloalkyl, $OR_8$, $N(R_9)R_{10}$, $C(O)OR_9$, $CON(R_9)R_{10}$ oder $N(R_9)COR_{11}$, oder worin zwei benachbarte Positionen an den aromatischen Ringen durch eine unsubstituierte oder durch Fluor ein- oder mehrfach substituierte Methylendioxy- oder Aethylendioxy-Gruppe überbrückt sind,

$R_8$ Wasserstoff oder $C_1$-$C_6$Alkyl, das unsubstituiert oder durch $C_1$-$C_3$-Alkoxy oder ein-oder mehrfach durch Halogen substituiert ist, oder $C_3$-$C_4$-Alkenyl, Propargyl, 3-Halogen-2-propinyl,

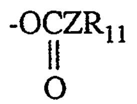 oder $COR_{11}$;

$R_9$ und $R_{10}$ unabhängig voneinander Wasserstoff oder $C_1$-$C_4$-Alkyl;

$R_{11}$ $C_1$-$C_4$-Alkyl;

Z Sauerstoff oder NH;

$R_6$ Wasserstoff, unsubstituiertes oder durch $C_1$-$C_4$-Alkoxy, Halogen und/oder Cyano ein-oder mehrfach substituiertes $C_1$-$C_6$-Alkyl, oder $C_3$-$C_6$-Cycloalkyl, $C_3$-$C_7$-Alkenyl, $C_3$-$C_7$-Alkinyl oder durch Halogen substituiertes $C_3$-$C_7$-Alkinyl oder

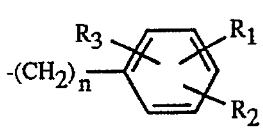 ;

n 0 oder 1;

$R_7$ X-$R_{12}$,

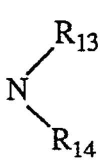 oder 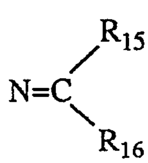 ;

X Sauerstoff oder Schwefel;

$R_{12}$ die Definitionen von $R_6$ oder

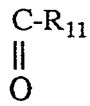;

$R_{13}$ und $R_{14}$ unabhängig voneinander die Definitionen von $R_{12}$, oder $R_{13}$ und $R_{14}$ gemeinsam eine $C_4$-$C_7$-Alkylenkette bilden, welche mit dem sie verbindenden Stickstoffatom einen unsubstituierten oder durch $C_1$-$C_4$-Alkyl mono- oder disubstituierten Heterocyclus bilden kann, wobei eine der Methylengruppen aus dieser Kette durch O, S oder $NR_9$ ersetzt sein kann;
$R_{15}$ und $R_{16}$ unabhängig voneinander die Definitionen von $R_6$, oder $R_{15}$ und $R_{16}$ gemeinsam eine $C_4$-$C_7$-Alkylenkette bilden, wobei diese durch $C_1$-$C_4$-Alkyl mono- oder disubstituiert sein kann und/oder eine der Methylengruppe aus dieser Kette durch O, S oder $NR_9$ ersetzt sein kann;
einschliesslich der Säureadditionsalze der Verbindungen der Formel I.

Unter dem Begriff Alkyl selbst oder als Bestandteil eines anderen Substituenten sind je nach der Zahl der angegebenen Kohlenstoffatome beispielsweise folgende Gruppen zu verstehen: Methyl, Ethyl, Propyl, Butyl, Pentyl, Hexyl usw., sowie ihre Isomeren, wie z.B. Isopropyl, Isobutyl, tert.-Butyl, Isopentyl usw.. Cycloalkyl kann beispielsweise Cyclopropyl, Cyclopentyl oder Cyclohexyl bedeuten. Haloalkyl steht für einen einfach bis perhalogenierten Alkylsubstituenten, wie z.B. $CH_2Cl$, $CHCl_2$, $CCl_3$, $CH_2Br$, $CHBr_2$, $CBr_3$, $CH_2F$, $CHF_2$, $CF_3$, $CCl_2F$, $CCl_2$-$CHCl_2$, $CH_2CH_2F$, $CJ_3$ usw. Unter Halogen soll hier und im folgenden Fluor, Chlor, Brom oder Jod, vorzugsweise Fluor oder Chlor verstanden werden.

Von den durch $R_{13}$ und $R_{14}$ gemeinsam mit dem anliegenden Stickstoffatom gebildeten unsubstituierten oder substituierten Heterocyclen sind folgende Reste besonders hervorzuheben:

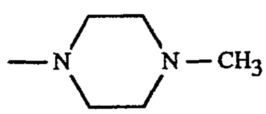, 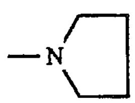, 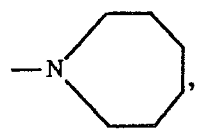, 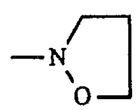, 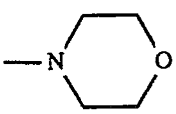 oder

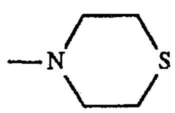.

Die Erfindung betrifft sowohl die freien Verbindungen der Formel I als auch deren Additionssalze mit anorganischen und organischen Säuren.

Erfindungsgemässe Salze sind insbesondere Additionssalze mit unbedenklichen anorganischen oder organischen Säuren, beispielsweise Halogenwasserstoffsäuren, z.B. Chlor-, Brom- oder Jodwasserstoffsäure, Schwefelsäure, Phosphorsäure, phosphorige Säure, Salpetersäure, oder organischen Säuren wie Essigsäure, Trifluoressigsäure, Trichloressigsäure, Propionsäure, Glycolsäure, Thiocyansäure, Milchsäure, Bernsteinsäure, Zitronensäure, Benzoesäure, Zimtsäure, Oxalsäure, Ameisensäure, Benzolsulfonsäure, p-Toluolsulfonsäure, Methansulfonsäure, Salicylsäure, p-Aminosalicylsäure, 2-Phenoxybenzoesäure, 2-Acetoxybenzoesäure oder 1,2-Naphthalin-disulfonsäure.

Die Verbindungen der Formel I sind unter Normalbedingungen stabile Oele, Harze oder überwiegend kristalline Feststoffe, die sich durch äusserst wertvolle mikrobizide Eigenschaften auszeichnen. Sie lassen sich beispielsweise auf dem Agrarsektor oder verwandten Gebieten präventiv und kurativ zur Bekämpfung pflanzenschädigender Mikroorganismen einsetzen. Die erfindungsgemässen Wirkstoffe der Formel I zeichnen sich in weiten Anwendungskonzentrationen durch eine hohe fungizide Aktivität und problemlose Anwendung aus. Es wird keine Schädigung der behandelten Pflanzen beobachtet.

Aufgrund ihrer besonderen pflanzenschützenden Eigenschaften lassen sich die Verbindungen der Formel I in folgende Gruppen einteilen:

1. Verbindungen der Formel I, worin bedeuten:

$R_1$, $R_2$, $R_3$, $R_4$ und $R_5$ unabhängig voneinander Wasserstoff, Halogen, Nitro, Cyano, unsubstituiertes oder durch Halogen und/oder $C_1$-$C_3$-Alkoxy ein- oder mehrfach substi tuiertes $C_1$-$C_6$-Alkyl, oder $C_3$-$C_6$-Cycloalkyl, $OR_8$ oder $N(R_9)R_{10}$; oder worin zwei benachbarte Positionen an den aromatischen Ringen durch eine unsubstituierte oder durch Fluor ein- oder mehrfach substituierte Methylendioxy- oder Aethylendioxy-Gruppe überbrückt sind;

$R_8$ Wasserstoff oder $C_1$-$C_6$Alkyl, das unsubstituiert oder durch $C_1$-$C_3$-Alkoxy oder ein-oder mehrfach

durch Halogen substituiert ist, oder $C_3$-$C_4$-Alkenyl, Propargyl oder 3-Halogen-2-propinyl;
$R_9$ und $R_{10}$ unabhängig voneinander Wasserstoff oder $C_1$-$C_4$-Alkyl;
$R_6$ Wasserstoff, unsubstituiertes oder durch $C_1$-$C_4$-Alkoxy, Halogen und/oder Cyano ein-oder mehrfach substituiertes $C_1$-$C_6$-Alkyl, oder $C_3$-$C_6$-Cycloalkyl, $C_3$-$C_7$-Alkenyl, $C_3$-$C_7$-Alkinyl oder durch Halogen substituiertes $C_3$-$C_7$Alkinyl;
$R_7$ X-$R_{12}$,

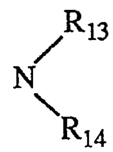 oder 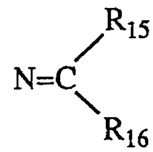 ;

X Sauerstoff oder Schwefel;
$R_{12}$ Wasserstoff, unsubstituiertes oder durch $C_1$-$C_4$-Alkoxy, Halogen und/oder Cyano ein-oder mehrfach substituiertes $C_1$-$C_6$-Alkyl, oder $C_3$-$C_6$-Cycloalkyl, $C_3$-$C_7$-Alkenyl, $C_3$-$C_7$-Alkinyl oder durch Halogen substituiertes $C_3$-$C_7$-Alkinyl oder

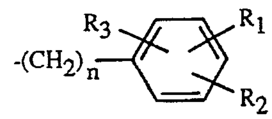 oder 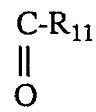 ;

$R_{11}$ $C_1$-$C_4$-Alkyl;
n 0 oder 1;
$R_{13}$ und $R_{14}$ unabhängig voneinander die Definitionen von $R_{12}$, oder $R_{13}$ und $R_{14}$ gemeinsam eine $C_4$-$C_7$-Alkylenkette bilden, welche mit dem sie verbindenden Stickstoffatom einen unsubstituierten oder durch $C_1$-$C_4$-Alkyl mono- oder disubstituierten Heterocyclus bilden kann, wobei eine der Methylengruppen aus dieser Kette durch O, S oder $NR_9$ ersetzt sein kann;
$R_{15}$ und $R_{16}$ unabhängig voneinander Wasserstoff, unsubstituiertes oder durch $C_1$-$C_4$-Alkoxy, Halogen und/oder Cyano ein- oder mehrfach substituiertes $C_1$-$C_6$-Alkyl, oder $C_3$-$C_6$-Cycloalkyl, $C_3$-$C_7$-Alkenyl, $C_3$-$C_7$-Alkinyl oder durch Halogen substituiertes $C_3$-$C_7$-Alkinyl oder

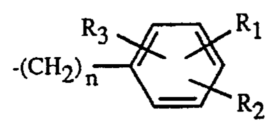 ;

oder $R_{15}$ und $R_{16}$ gemeinsam eine $C_4$-$C_7$-Alkylenkette bilden, wobei diese durch $C_1$-$C_4$ Alkyl mono- oder disubstituiert sein kann und/oder eine der Methylengruppe aus dieser Kette durch O, S oder $NR_9$ ersetzt sein kann;
einschliesslich der Säureadditionsalze der Verbindungen der Formel I.
2. Verbindungen der Formel I, worin bedeuten:
$R_1$, $R_2$, $R_3$, $R_4$ und $R_5$ unabhängig voneinander Wasserstoff, Halogen, Nitro, Cyano, unsubstituiertes oder durch Halogen und/oder $C_1$-$C_3$-Alkoxy ein- oder mehrfach substituiertes $C_1$-$C_6$-Alkyl, oder $C_3$-$C_6$-Cycloalkyl, $OR_8$ oder $N(R_9)R_{10}$; oder worin zwei benachbarte Positionen an den aromatischen Ringen durch eine unsubstituierte oder durch Fluor ein- oder zweifach substituierte Methylendioxy-Gruppe überbrückt sind,
$R_8$ Wasserstoff oder $C_1$-$C_6$Alkyl, das unsubstituiert oder durch $C_1$-$C_3$-Alkoxy oder ein-oder mehrfach durch Halogen substituiert ist, oder $C_3$-$C_4$-Alkenyl, Propargyl oder 3-Halogen-2-propinyl;
$R_9$ und $R_{10}$ unabhängig voneinander Wasserstoff oder $C_1$-$C_4$-Alkyl;
$R_6$ Wasserstoff, unsubstituiertes oder durch $C_1$-$C_2$-Alkoxy, Halogen und/oder Cyano ein-oder mehrfach substituiertes $C_1$-$C_4$-Alkyl, oder $C_3$-$C_6$-Cycloalkyl, $C_3$-$C_5$-Alkenyl, $C_3$-$C_5$-Alkinyl oder 3-Halogen-2-propinyl;
$R_7$ X-$R_{12}$,

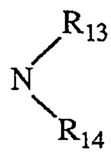 oder 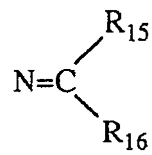 ;

X Sauerstoff oder Schwefel;

$R_{12}$ Wasserstoff, unsubstituiertes oder durch $C_1$-$C_4$-Alkoxy, Halogen und/oder Cyano ein-oder mehrfach substituiertes $C_1$-$C_6$-Alkyl, oder $C_3$-$C_6$-Cycloalkyl, $C_3$-$C_7$-Alkenyl, $C_3$-$C_7$-Alkinyl, 3-Halogen-2-propinyl oder

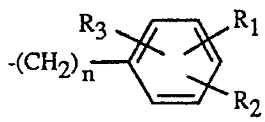 ;

n 0 oder 1;

$R_{13}$ und $R_{14}$ unabhängig voneinander die Definitionen von $R_{12}$ oder $R_{13}$ und $R_{14}$ gemeinsam eine $C_4$-$C_7$-Alkylenkette bilden, welche mit dem sie verbindenden Stickstoffatom einen unsubstituierten oder durch $C_1$-$C_4$-Alkyl mono- oder disubstituierten Heterocyclus bilden kann, wobei eine der Methylengruppen aus dieser Kette durch O oder S ersetzt sein kann;

$R_{15}$ und $R_{16}$ unabhängig voneinander Wasserstoff, unsubstituiertes oder durch $C_1$-$C_4$-Alkoxy, Halogen und/oder Cyano ein- oder mehrfach substituiertes $C_1$-$C_6$-Alkyl, oder $C_3$-$C_6$-Cycloalkyl, $C_3$-$C_7$-Alkenyl, $C_3$-$C_7$-Alkinyl, 3-Halogen-2-propinyl oder

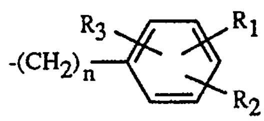 ;

oder $R_{15}$ und $R_{16}$ gemeinsam eine $C_4$-$C_7$-Alkylenkette bilden, wobei diese durch $C_1$-$C_4$-Alkyl mono- oder disubstituiert sein kann und/oder eine der Methylengruppe aus dieser Kette durch O oder S ersetzt sein kann;

einschliesslich der Säureadditionsalze der Verbindungen der Formel I.

3. Verbindungen der Formel I, worin bedeuten:

$R_1$, $R_2$, $R_3$, $R_4$ und $R_5$ unabhängig voneinander Wasserstoff, Halogen, Nitro, Cyano, unsubstituiertes oder durch Halogen und/oder $C_1$-$C_3$-Alkoxy ein- oder mehrfach substituiertes $C_1$-$C_6$-Alkyl, oder $C_3$-$C_6$-Cycloalkyl, $OR_8$ oder $N(R_9)R_{10}$; oder worin zwei benachbarte Positionen an den aromatischen Ringen durch eine unsubstituierte oder durch Fluor ein- oder zweifach substituierte Methylendioxy-Gruppe überbrückt sind,

$R_8$ Wasserstoff oder $C_1$-$C_6$Alkyl, das unsubstituiert oder durch $C_1$-$C_3$-Alkoxy oder ein-oder mehrfach durch Halogen substituiert ist, oder $C_3$-$C_4$-Alkenyl, Propargyl oder 3-Halogen-2-propinyl;

$R_9$ und $R_{10}$ unabhängig voneinander Wasserstoff oder $C_1$-$C_4$-Alkyl;

$R_6$ Wasserstoff, unsubstituiertes oder durch $C_1$-$C_2$-Alkoxy, Halogen und/oder Cyano ein-oder mehrfach substituiertes $C_1$-$C_4$-Alkyl, oder $C_3$-$C_6$-Cycloalkyl, $C_3$-$C_5$-Alkenyl, $C_3$-$C_5$-Alkinyl oder 3-Halogen-2-propinyl;

$R_7$ X-$R_{12}$,

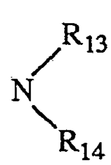 oder 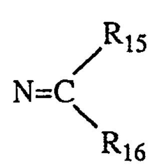 ;

X Sauerstoff oder Schwefel;

$R_{12}$ Wasserstoff, unsubstituiertes oder durch $C_1$-$C_2$-Alkoxy, Halogen und/oder Cyano ein-oder mehrfach substituiertes $C_1$-$C_4$-Alkyl, oder $C_3$-$C_6$-Cycloalkyl, $C_3$-$C_5$-Alkenyl, $C_3$-$C_5$-Alkinyl oder 3-Halogen-2-propi-

nyl;

$R_{13}$ und $R_{14}$ unabhängig voneinander die Definitionen von $R_{12}$, oder $R_{13}$ und $R_{14}$ gemeinsam eine $C_4$-$C_7$-Alkylenkette bilden, welche mit dem sie verbindenden Stickstoffatom einen unsubstituierten oder durch $C_1$-$C_4$-Alkyl mono- oder disubstituierten Heterocyclus bilden kann, wobei eine der Methylengruppen aus dieser Kette durch O oder S ersetzt sein kann;

$R_{15}$ und $R_{16}$ unabhängig voneinander Wasserstoff, unsubstituiertes oder durch $C_1$-$C_2$-Alkoxy, Halogen und/oder Cyano ein- oder mehrfach substituiertes $C_1$-$C_4$-Alkyl, oder $C_3$-$C_6$-Cycloalkyl, $C_3$-$C_5$-Alkenyl, $C_3$-$C_5$-Alkinyl oder 3-Halogen-2-propinyl; oder $R_{15}$ und $R_{16}$ gemeinsam eine $C_4$-$C_7$-Alkylenkette bilden, wobei diese durch $C_1$-$C_4$-Alkyl mono- oder disubstituiert sein kann und/oder eine der Methylengruppen aus dieser Kette durch O oder S ersetzt sein kann;

einschliesslich der Säureadditionssalze der Verbindungen der Formel I.

4. Verbindungen der Formel I, worin bedeuten:

$R_1$, $R_2$ und $R_3$ unabhängig voneinander Wasserstoff, Halogen, unsubstituiertes oder durch Halogen und/oder $C_1$-$C_2$-Alkoxy ein- oder mehrfach substituiertes $C_1$-$C_3$-Alkyl, $OR_8$, $N(R_9)R_{10}$oder worin zwei benachbarte Positionen an den aromatischen Ringen durch Difluormethylendioxy überbrückt sind;

$R_8$ Wasserstoff oder $C_1$-$C_3$ Alkyl, das unsubstituiert oder durch $C_1$-$C_3$-Alkoxy oder ein-oder mehrfach durch Halogen substituiert ist;

$R_4$ und $R_5$ unabhängig voneinander Wasserstoff, Halogen, $NO_2$ oder $C_1$-$C_3$-Alkoxy, oder worin zwei benachbarte Positionen an den aromatischen Ringen durch eine unsubstituierte oder durch Fluor ein- oder zweifach substituierte Methylendioxy-Gruppe überbrückt sind;

$R_9$ und $R_{10}$ unabhängig voneinander Wasserstoff oder $C_1$-$C_4$-Alkyl;

$R_6$ Wasserstoff, unsubstituiertes oder durch $C_1$-$C_2$-Alkoxy, Halogen und/oder Cyano substituiertes $C_1$-$C_4$-Alkyl, oder 2-Allyl, 2-Propinyl oder 3-Jod-2-propinyl;

$R_7$ X-$R_{12}$,

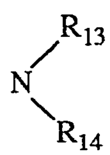 oder 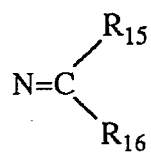 ;

X Sauerstoff oder Schwefel;

$R_{12}$ Wasserstoff, $C_1$-$C_4$-Alkyl, 2-Allyl oder 2-Propinyl;

$R_{13}$ und $R_{14}$ unabhängig voneinander die Definitionen von $R_{12}$, oder $R_{13}$ und $R_{14}$ gemeinsam eine $C_4$-$C_7$-Alkylenkette bilden, welche mit dem sie verbindenden Stickstoffatom einen unsubstituierten Heterocyclus bilden kann, wobei eine der Methylengruppen aus dieser Kette durch O oder S ersetzt sein kann;

$R_{15}$ und $R_{16}$ unabhängig voneinander die Definitionen von $R_{13}$ oder $R_{14}$.

5. Verbindungen der Formel I, worin bedeuten:

$R_1$, $R_2$ und $R_3$ unabhängig voneinander Wasserstoff, Halogen, $CF_3$, $OR_8$ oder $N(R_9)R_{10}$;

$R_8$ Wasserstoff, $C_1$-$C_3$-Alkyl oder $CHF_2$;

$R_9$ und $R_{10}$ unabhängig voneinander Wasserstoff oder $C_1$-$C_3$-Alkyl;

$R_4$ und $R_5$ unabhängig voneinander Wasserstoff, Halogen oder Methoxy;

$R_6$ Wasserstoff, $C_1$-$C_4$-Alkyl oder $C_1$-$C_3$-Cyanoalkyl;

$R_7$ X-$R_{12}$,

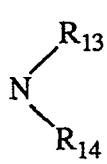 oder 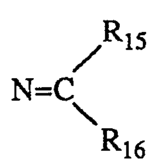 ;

X Sauerstoff oder Schwefel;

$R_{12}$ Wasserstoff oder $C_1$-$C_4$-Alkyl;

$R_{13}$, $R_{14}$, $R_{15}$ und $R_{16}$ unabhängig voneinander die Definitionen von $R_{12}$, oder $R_{13}$ und $R_{14}$ und/oder $R_{15}$ und $R_{16}$ gemeinsam eine $C_4$-$C_5$-Alkylenkette bilden, wobei eine der Methylengruppe durch O oder S ersetzt sein kann.

Folgende Verbindungen zeichnen sich durch besonders vorteilhafte pflanzenschützende Eigenschaften aus:
1-(3,4-Dimethoxyphenyl)-2-naphthoesäure-N-methyl-N-methoxy-amid;
1-(3,4-Dimethoxyphenyl)-2-naphthoesäure-N-methyl-N-ethoxy-amid;
1-(3,4-Dimethoxyphenyl)-2-naphthoesäure-N-ethyl-N-methoxy-amid;
1-(3,4-Dimethoxyphenyl)-2-naphthoesäure-N-ethyl-N-ethoxy-amid;
1,2-Dimethyl-1-[1-(3,4-dimethoxyphenyl)-2-naphthoyl]-hydrazin;
1,2,2-Trimethyl-1-[1-(3,4-dimethoxyphenyl)-2-naphthoyl]-hydrazin;
1-Methyl-1-[1-(3,4-dimethoxyphenyl)-2-naphthoyl]-hydrazono-isopropan.

Die Verbindungen der Formel I werden wie folgt hergestellt:
A) Umsetzung einer Aminoverbindung der Formel II

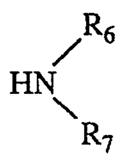

(II)

mit einem Naphthoesäurederivat der Formel III

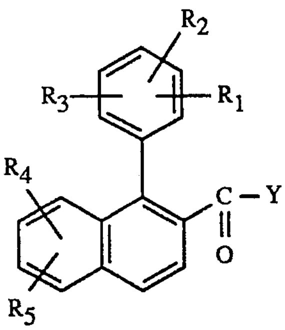

(III),

worin Y OH, Halogen oder $C_1$-$C_4$-Alkoxy bedeutet, zu einer Verbindung der Formel Ia

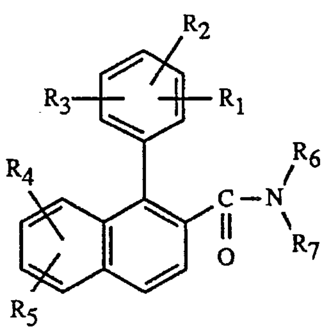

(Ia),

worin $R_1$-$R_7$ die unter Formel I angegebenen Bedeutungen besitzen, bei Temperaturen von -25° bis 150° C, vorzugsweise von -10° C bis zur Siedetemperatur des Reaktionsgemisches, in Gegenwart eines säureaktivierenden und/oder eines wasserentziehenden Mittels in einem reaktionsinerten Lösungsmittel oder Lösungsmittelgemisch.
B) Umsetzung einer Verbindung der Formel Ia mit Phosphorpentasulfid zu einer Verbindung der Formel Ib

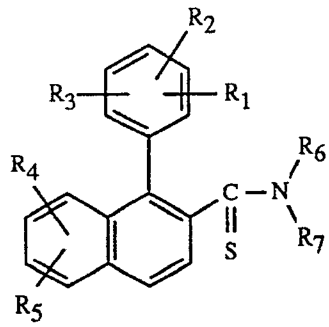

(Ib),

worin $R_1$-$R_7$ die unter Formel I angegebenen Bedeutungen besitzen, bei Temperaturen von 0° bis 120° C, in inerten Lösungsmitteln, wobei das Phosphorpentasulfid vorteilhaft in Gegenwart von $K_2S$ oder $K_2(S_x)$ eingesetzt werden kann.

C) Umsetzung einer Verbindung der Formel Ic

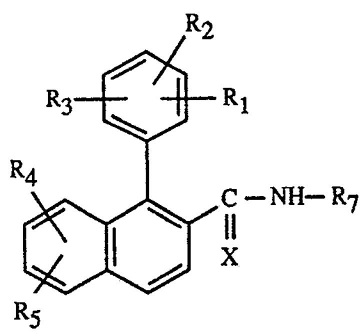

(Ic),

worin $R_1$-$R_7$ und X die unter Formel I angegebenen Bedeutungen darstellen, mit einer Verbindung der Formel IV

$Q$-$R_6$ (IV),

worin Q eine nucleophile Abgangsgruppe, wie z.B. Halogen, p-Tosyloxy, Trifluoracetyloxy, Benzolsulfonyloxy oder Mesyloxy, darstellt und die Bedeutung von $R_6$ unter Formel I angegeben ist, bei Temperaturen von 0° bis 220° C, bevorzugt 20° bis 170° C, in reaktionsinternen, vorzugsweise relativ polaren, Lösungsmitteln.

D) Umsetzung eines Ketons der Formel V

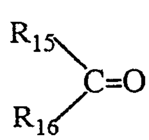

(V)

mit einer Hydrazinverbindung der Formel Ie

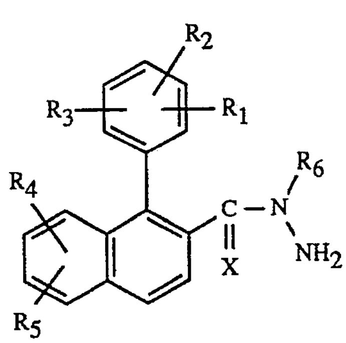

(Ie)

oder einem Salz davon zu einer Verbindung der Formel Id

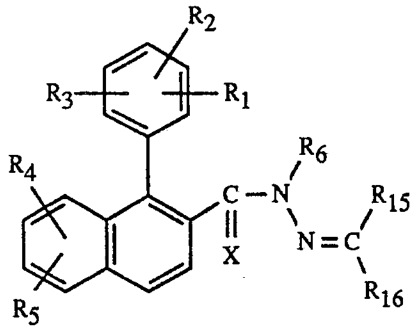

(Id),

worin $R_1$-$R_{16}$ und X die unter Formel I angegebenen Bedeutungen besitzen, bei Temperaturen von -20° bis 200° C, bevorzugt 20° C bis zum Siedepunkt des verwendeten Lösungsmittels, in einem reaktionsinerten Lösungsmittel oder in der Schmelze der Reaktionsteilnehmer, wobei das verwendete Lösungsmittel auch das an der Reaktion beteiligte Keton sein kann, sowie in Gegenwart eines Katalysators, wie z.B. einer geringen Menge einer anorganischen oder organischen Säure oder Base, oder ohne Katalysator.

E) Umsetzung einer Verbindung der Formel If

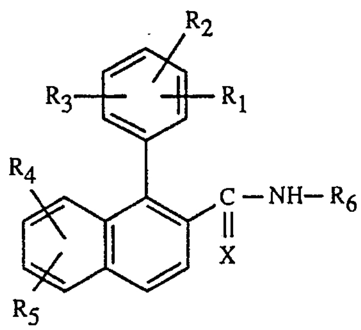

(If)

mit einer Schwefelverbindung der Formel VI

Hal-S-$R_{12}$ (VI)

zu einer Verbindung der Formel Ig

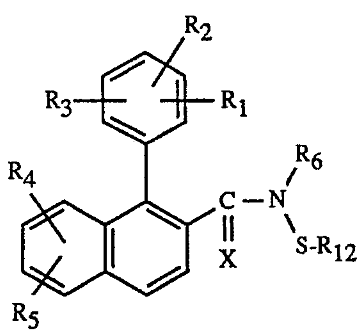

(Ig),

worin $R_1$-$R_{12}$ und X die unter Formel I angegebenen Bedeutungen besitzen, bei Temperaturen von -30° bis 150° C, vorzugsweise -20° bis 40° C, in reaktionsinerten Lösungsmitteln oder Gemischen davon in Gegenwart eines säurebindenden Mittels.

In dem vorstehend beschriebenen Verfahren gelangen folgende Reaktionshilfsmittel zur Anwendung.

Geeignete reaktionsinerte Lösungs- oder Verdünnungsmittel sind z.B. aliphatische und aromatische Kohlenwasserstoffe, wie Petrolether, Hexan, Benzol, Toluol oder Xylole; halogenierte Kohlenwasserstoffe, wie Chlorbenzol, Methylenchlorid, Chloroform oder Tetrachlorkohlenstoff; Nitrobenzol; Ether und etherartige Verbindungen, wie Dialkylether (Diethylether, Diisopropylether oder tert.-Butylmethylether) sowie Dioxan, Tetrahydrofuran; ferner Ethylacetat oder Methylacetat sowie Gemische derartiger Verbindungen untereinander.

Darüber hinaus sind weitere reaktionsinerte, jedoch relativ polare Lösungsmittel, z.B. N,N-Dimethylformamid, Hexamethylphosphorsäuretriamid (HMPT), N,N-Dimethylacetamid, Dimethylsulfoxid, Acetonitril, Benzonitril und andere zu nennen.

Als säurebindende Mittel kommen z.B. anorganische Basen, wie Hydroxide, Carbonate und Hydrogencarbonate von Alkali- und Erdalkalimetallen sowie die Hydride der Alkalimetalle in Frage, so z.B. NaOH, KOH, LiOH, $Mg(OH)_2$, $K_2CO_3$, $Na_2CO_3$, $KHCO_3$, $NaHCO_3$, $CaCO_3$ $MgCO_3$, $BaCO_3$ usw.. Ferner kommen organische Basen in Frage, so z.B. tertiäre Amine, wie Trialkylamine (Triethylamin, Diethylmethylamin, Tripropylamin, Dimethylanilin, Methylethylanilin, usw.), Pyridin und Pyridinbasen, wie 4-Dimethylaminopyridin oder 4-Pyrrolidylopyridin usw..

Als säureaktivierende und/oder wasserentziehende Mittel können beispielsweise Chlorameisensäureester wie Chlorameisensäureäthylester, oder Phosphorpentoxid, N,N′-Dicyclohexylcarbodiimid, N,N′-Carbonyldiimidazol oder N,N′-Thionyldiimidazol Anwendung finden.

Aus der Literatur sind verschiedentlich 1-Aryl-naphthoesäure-Derivate bekannt. So sind Amidderivate als pharmakologisch wirksame Substanzen in Ind. J. Pharm. Sci. 1985, 47 (1) 12-15; Chem. Abstr. 103, 25, 205882f (1985) beschrieben. Die europäische Patentanmeldung EP-251315 offenbart Ester- und Amidderivate mit humanmedizinischer Wirksamkeit. Darüber hinaus beschreibt die deutsche Offenlegungsschrift DE-OS 3710717 Arylnaphthoesäureamide mit bioziden, darunter auch fungiziden Eigenschaften. Diese Wirkstoffe konnten jedoch in der Praxis die an sie bei der Bekämpfung von pflanzenschädigenden Pilzen gestellten Forderungen vor allem bei niedrigen Anwendungskonzentrationen nicht in ausreichendem Masse erfüllen.

Es wurde nun überraschend festgestellt, dass die erfindungsgemässen Verbindungen der Formel I ein sehr vorteilhaftes, die praktischen Bedürfnisse gut befriedigendes biozides Wirkungsspektrum gegen schädliche Mikroorganismen, insbesondere gegen phytopathogene Pilze und Bakterien, aufweisen. Sie besitzen sehr vorteilhafte kurative, systemische und insbesondere präventive Eigenschaften und lassen sich zum Schutz von zahlreichen Kulturpflanzen einsetzen. Mit dem Wirkstoff der Formel I können an Pflanzen oder an Pflanzenteilen (Früchte, Blüten, Laubwerk, Stengel, Knollen, Wurzeln) von unterschiedlichen Nutzkulturen die auftretenden Schädlinge eingedämmt oder vernichtet werden, wobei auch später zuwachsende Pflanzenteile von phytopathogenen Mikroorganismen verschont bleiben.

Die erfindungsgemässen Wirkstoffe sind beispielsweise gegen die den folgenden Klassen angehörenden phytopathogenen Pilze wirksam: Ascomycetes z.B. Erysiphe, Sclerotinia, Fusarium, Monilinia, Helminthosporium; Basidiomycetes wie z.B. Puccinia, Tilletia, Rhizoctonia; sowie insbesondere die der Klasse der Phycomycetes angehörenden Oomycetes wie Phytophthora, Plasmopara, Peronospora, Pythium. Als Pflanzenschutzmittel können die Verbindungen der Formel I auch gegen wichtige Schadpilze aus der Familie der Fungi imperfecti eingesetzt werden, so z.B. gegen Cercospora, Pyricularia und Botrytis. Ueberdies wirken die Verbindungen systemisch. Darüber hinaus lassen sich Verbindungen der Formel I erfolgreich zum Schutz verderblicher Waren pflanzlicher oder tierischer Herkunft einsetzen. Sie bekämpfen Schimmelpilze wie Penicillium, Aspergillus, Rhizopus, Fusarium, Helminthosporium, Nigrospora und Alternaria sowie Bakterien wie Buttersäurebakterien und Hefen wie Candida. Diese Wirksubstanzen zeigen ferner hervorragende Wirkung gegen Boden- und Samen-bürtige Pilze.

Die Wirkstoffe der Formel I können ferner auch als Beizmittel zur Behandlung von Saatgut (Früchte, Knollen, Körner) und Pflanzenstecklingen zum Schutz vor Pilzinfektionen sowie gegen im Erdboden auftretende phytopathogene Pilze eingesetzt werden, wobei sie sich insbesondere als Getreidebeizmittel in der Bekämpfung von Pilzorganismen, wie beispielsweise Fusarium-, Helminthosporium und Tilletia-Arten, auszeichnen.

Die Erfindung betrifft somit auch mikrobizide Mittel sowie die Verwendung der Verbindungen der Formel I zur Bekämpfung phytopathogener Mikroorganismen, insbesondere pflanzenschädigender Pilze bzw. die präventive Verhütung eines Befalls an Pflanzen und an Vorräten pflanzlicher oder tierischer Herkunft.

Als Zielkulturen für den Pflanzenschutz gelten im Rahmen dieser Erfindung beispielsweise folgende Pflanzenarten: Getreide: (Weizen, Gerste, Roggen, Hafer, Reis, Sorghum und Verwandte); Rüben: (Zucker- und Futterrüben): Kern-, Stein- und Beerenobst: (Aepfel, Birnen, Pflaumen, Pfirsiche, Mandeln, Kirschen, Erd-, Him- und Brombeeren); Hülsenfrüchte: (Bohnen, Linsen, Erbsen, Soja): Oelkulturen: (Raps, Senf, Mohn, Oliven, Sonnenblumen, Kokos, Rizinus, Kakao, Erdnüssen); Gurkengewächse: (Kürbis, Gurken, Melonen); Fasergewächse: (Baumwolle, Flachs, Hanf, Jute); Citrusfrüchte: (Orangen, Zitronen, Pampelmusen, Mandarinen); Gemüsesorten: (Spinat, Kopfsalat, Spargel, Kohlarten, Möhren, Zwiebeln, Tomaten, Kartoffeln, Paprika); Lorbeergewächse: (Avocado, Cinnamonum, Kampfer) oder Pflanzen wie Mais, Tabak, Nüsse, Kaffee, Zuckerrohr, Tee, Weinreben, Hopfen, Bananen-und Naturkautschukgewächse sowie Zierpflanzen (Compositen).

Wirkstoffe der Formel I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelement-Vermittler oder andere das Pflanzenwachstum beeinflussende Präparate sein. Es können aber auch selektive Herbizide, Insektizide, Fungizide, Bakterizide, Nematizide, Molluskizide oder Gemische mehrerer dieser Präparate sein, zusammen mit gegebenenfalls weiteren in der Formulierungstechnik üblichen Träger stoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Ein bevorzugtes Verfahren zum Aufbringen eines Wirkstoffes der Formel I bzw. eines (agro)chemischen Mittels, das mindestens einen dieser Wirkstoffe enthält, ist das Aufbringen auf das Blattwerk (Blattapplikation). Anzahl der Applikationen und Aufwandmenge richten sich dabei nach dem Befallsdruck für den entsprechenden Erreger (Pilzsorte). Die Wirkstoffe der Formel I können aber auch über den Erdboden durch das Wurzelwerk in die Pflanze gelangen (systemische Wirkung), indem man den Standort der Pflanze mit einer flüssigen Zubereitung tränkt oder die Substanzen in fester Form in den Boden einbringt, z.B. in Form von Granulat (Bodenapplikation). Die Verbindungen der Formel I können aber auch auf Samenkörner aufgebracht werden (Coating), indem man die Körner entweder mit einer flüssigen Zübereitung des Wirkstoffs tränkt oder sie mit einer festen Zübereitung beschichtet. Darüber hinaus sind in besonderen Fällen weitere Applikationsarten möglich, so z.B. die gezielte Behandlung der Pflanzenstengel oder der Knospen.

Die Verbindungen der Formel I werden dabei in unveränderter Form oder vorzugsweise zusammen mit den in der Formulierungstechnik üblichen Hilfsmittel eingesetzt und werden daher z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 50 g bis 5 kg Aktivsubstanz (AS) je ha; bevorzugt 100 g bis 2 kg AS/ha, insbesondere bei 200 g bis 600 g AS/ha.

Die Formulierungen d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Träger stoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethylenglykolmonomethylether, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle oder Sojaöl, oder Wasser.

Als feste Trägerstoffe, z.B. für Stäubemittel und dispergierbare Pulver, lassen sich Calcit, Talkum, Kaolin, Montmorillonit oder Attapulgit, hochdisperse Kieselsäure oder saugfähige Polymerisate verwenden. Als gekörnte, adsorptive Granulatträger kommen Bimsstein, Ziegelbruch, Sepiolit oder Bentonit, als nichtsorptive Träger z.B. Calcit oder Dolomit in Frage. Es können auch zerkleinerte Pflanzenrückstände verwendet werden.

Als oberflächenaktive Verbindungen kommen je nach Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Weitere in der Formulierungstechnik gebräuchliche Tenside sind dem Fachmann bekannt oder können der einschlägigen Fachliteratur entnommen werden.

Die agrochemischen Zübereitungen enthalten in der Regel 0,1 bis 99 %, insbesondere 0,1 bis 95 % Wirkstoff der Formel I, 99,9 bis 1 %, insbesondere 99,9 bis 5 % eines festen oder flüssigen Zusatzstoffes und 0 bis 25 %, insbesondere 0,1 bis 25 % eines Tensides.

Während als Handelsware eher konzentrierte Mittel bevorzugt werden, verwendet der Endverbraucher in der Regel verdünnte Mittel.

Derartige (agro)chemische Mittel sind ein Bestandteil der vorliegenden Erfindung.

Die nachfolgenden Beispiele dienen der Illustration der Erfindung, ohne dieselbe einzuschränken.

1. Herstellungsbeispiele

Beispiel 1: Herstellung von 1-(3,4-Dimethoxyphenyl)-2-naphthoesäure-N-methyl-N-methoxy-amid

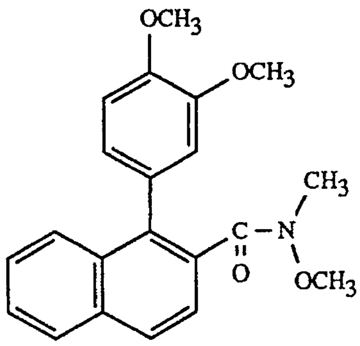

7,0 g 1-(3,4-Dimethoxyphenyl)-2-naphthoesäure werden in 30 ml Toluol vorgelegt und mit 2,8 g Thionylchlorid versetzt. Die Reaktionsmischung wird zum Sieden erhitzt und 2 Std. unter Rückfluss gerührt. Anschliessend wird unter Vakuum das Lösungsmittel abgedampft und der Rückstand in 30 ml Dimethylformamid aufgenommen. Die so erhaltene Lösung wird innert 3/4 Std. bei 0 bis 5° C zu einer Suspension von 2,2 g N,O-Dimethylhydroxylamin-Hydrochlorid und 4,7 g Triethylamin in 20 ml Dimethylformamid getropft. Anschliessend lässt man die Temperatur des Reaktionsgemisches auf Raumtemperatur ansteigen und rührt 3 Std. bei dieser Temperatur nach.

Zur Aufarbeitung wird das Reaktionsgemisch auf 400 ml Wasser gegeben und 2 mal mit je 150 ml Ethylacetat extrahiert. Die organischen Phasen werden vereinigt, 3 mal mit je 150 ml Wasser gewaschen, über Natriumsulfat getrocknet, filtriert und das Lösungsmittel unter Vakuum entfernt. Der verbleibende Rückstand wird zur Reinigung mittels Ethylacetat über eine Kieselgelsäule chromatographiert. Die Titelverbindung wird in Form weisser Kristalle erhalten; Smp. 107-108° C.

Beispiel 2: Herstellung von 1-(3,4-Dimethoxyphenyl)-2-naphthoesäure-N-ethoxy-amid

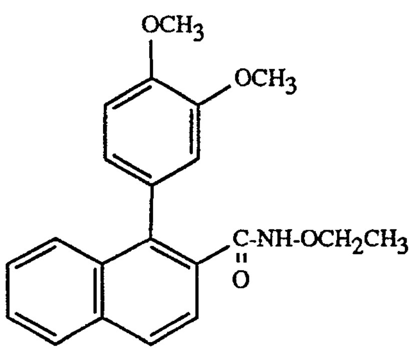

9,6 g 1-(3,4-Dimethoxyphenyl)-2-naphthoesäure und 3,7 g Thionylchlorid werden 2,5 Stunden in 40 ml Toluol unter Rückfluss gerührt. Anschliessend wird das Lösungsmittel abgedampft und der Rückstand in 40 ml Tetrahydrofuran gelöst. Diese Lösung wird innerhalb 1,5 Stunden bei 0 bis 5° C zu einem Gemisch von 3,05 g 0-Aethylhydroxylamin-Hydrochlorid und 6,4 g Triethylamin in 25 ml Dimethylformamid getropft. Anschliessend wird 16 Stunden bei Raumtemperatur gerührt, mit ca. 100 ml Essigsäureethylester verdünnt und das Reaktionsgemisch 3 mal mit je 150 ml Wasser gewaschen.

Die organische Phase wird über Natriumsulfat getrocknet, filtriert und das Lösungsmittel verdampft. Der feste Rückstand wird in Diethylether digeriert. Die Titelverbindung wird dabei in Form weisser Kristalle erhalten. Smp.: 172- 173° C.

Beispiel 3: Herstellung von 1-(3,4-Dimethoxyphenyl)-2-naphthoesäure-N-methyl-N-ethoxy-amid

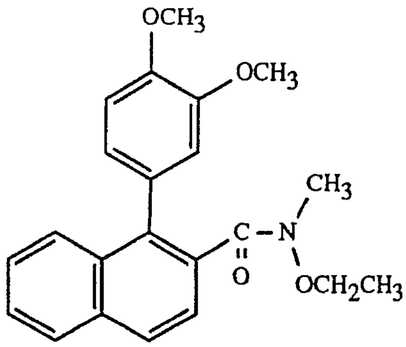

5,8 g des im Beispiel 2 hergestellten 1-(3,4-Dimethoxyphenyl)-2-naphthoesäure-N-ethoxy-amids und 0,7 g Natriumhydrid (55%-ig in Mineralöl) werden 1,5 Stunden bei Raumtemperatur in 110 ml Dimethylformamid gerührt. Anschliessend werden innerhalb 10 Minuten 2,6 g Methyljodid zugetropft (die Temperatur steigt dabei von 23° C auf 27° C an). Nach beendeter Zugabe wird 4 Stunden bei Raumtemperatur gerührt, dann das Reaktionsgemisch in 500 ml Wasser gegossen und 3 mal mit je 100 ml Ethylacetat extrahiert. Die organischen Phasen werden vereinigt, über Natriumsulfat getrocknet, filtriert und das Lösungsmittel am Vakuum verdampft. Der feste Rückstand wird zur Reinigung mittels Diethylether über eine Kieselgelsäule chromatographiert. Die Titelverbindung wird in Form weisser Kristalle erhalten. Smp.: 115-116° C.

Tabelle 1:

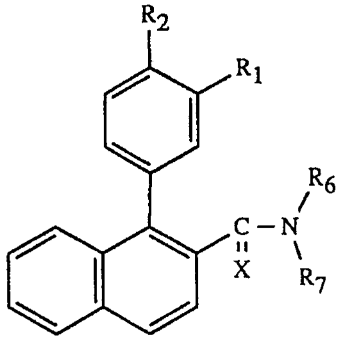

| Nr. | $R_1$ | $R_2$ | X | $R_6$ | $R_7$ | physikal. Daten |
|---|---|---|---|---|---|---|
| 1.1. | $OCH_3$ | $OCH_3$ | O | $CH_3$ | $OCH_3$ | Smp. 107-108°C |
| 1.2. | $OCH_3$ | $OCH_3$ | O | H | $OCH_3$ | Smp. 157-158°C |
| 1.3. | $OCH_3$ | H | S | $CH_3$ | $OCH_3$ | |
| 1.4. | Cl | $OCH_3$ | O | $CH_3$ | $OCH_3$ | |
| 1.5. | $OCH_3$ | $OCH_3$ | O | $CH_3$ | $NH-CH_3$ | Smp. 128-130°C |
| 1.6. | $OCH_3$ | $OCH_3$ | O | $CH_3$ | $S-CH_3$ | |
| 1.7. | $OCH_3$ | $OCH_3$ | O | $CH_2CH_3$ | $S-CH_3$ | |
| 1.8. | $OCH_3$ | $OCH_3$ | O | $CH_2CH_3$ | $OCH_3$ | Smp. 104-105°C |
| 1.9. | $OCH_3$ | $OCH_3$ | O | $CH_3$ | $S-CH_2CH_3$ | |
| 1.10. | $OCH_3$ | $OCH_3$ | O | $CH_2CH_3$ | $N(CH_3)_2$ | Smp. 159-161°C |
| 1.11. | $OCH_3$ | H | O | $CH_3$ | $OCH_3$ | |
| 1.12. | $OCH_3$ | $OCH_3$ | O | $CH_3$ | $NH_2$ | |
| 1.13. | $OCH_3$ | $OCH_3$ | O | $CH_3$ | $-S-CCl_2F$ | |
| 1.14. | $OCH_3$ | $OCH_3$ | O | $CH(CH_3)_2$ | $OCH_3$ | Smp. 98-100°C |
| 1.15. | Cl | $OCH_3$ | O | $CH_2CH_3$ | $OCH_3$ | |

| Nr. | $R_1$ | $R_2$ | X | $R_6$ | $R_7$ | physikal. Daten |
|---|---|---|---|---|---|---|
| 1.16. | $OCH_3$ | $OCH_3$ | S | $CH_3$ | $NH-CH_3$ | |
| 1.17. | $OCH_3$ | $OCH_3$ | S | $CH_3$ | $SCH_3$ | |
| 1.18. | $OCH_3$ | $OCH_3$ | O | $CH_3$ | $N(CH_3)CH_2CH_3$ | |
| 1.19. | $OCH_3$ | H | O | $CH(CH_3)_2$ | $OCH_3$ | |
| 1.20. | $OCH_3$ | $OCH_3$ | O | 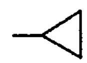 | $OCH_3$ | |
| 1.21. | $OCH_3$ | Cl | O | $CH_3$ | $SCH_3$ | |
| 1.22. | $OCH_3$ | $OCH_3$ | O | $C_2H_5$ | $OC_2H_5$ | Smp. 86 - 88°C |
| 1.23. | $OCH_3$ | $OCH_3$ | O | $CH_2CH=CH_2$ | $SCH_3$ | |
| 1.24. | $OCH_3$ | $OCH_3$ | O | 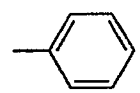 | $SCH_3$ | |
| 1.25. | $OCH_3$ | $OCH_3$ | O | $CH_2$ 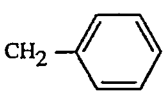 | $OCH_3$ | |
| 1.26. | $OCH_3$ | $OCH_3$ | O | $CH_2CH_3$ | $N=C(CH_3)_2$ | |
| 1.27. | $OCH_3$ | Cl | O | $CH_3$ | $OCH_3$ | |

| Nr. | $R_1$ | $R_2$ | X | $R_6$ | $R_7$ | physikal. Daten |
|---|---|---|---|---|---|---|
| 1.28. | $OCH_2CH_3$ | $OCH_2CH_3$ | O | $CH_3$ | $OCH_3$ | |
| 1.29. | $OCH_3$ | $OCH_3$ | O | $CH_2CH{=}CH_2$ | $OCH_3$ | |
| 1.30. | $OCH_3$ | $OCH_3$ | O | $C_2H_5$ | $SCH_2CH_3$ | |
| 1.31. | $OCH_3$ | $OCH_3$ | O | $CH_3$ | $N(CH_3)_2$ | Smp. 168-169°C |
| 1.32. | $OCH_3$ | $OCH_3$ | O | $C_2H_5$ | $SCCl_2F$ | |
| 1.33. | $OCH_3$ | $OCH_3$ | O | $CH_3$ | $OCH_2CH_3$ | Smp. 115-116°C |
| 1.34. | $OCH_3$ | $OC_2H_5$ | O | $CH_3$ | $OCH_3$ | |
| 1.35. | $OCH_3$ | $OCH_3$ | O | $CH_2CH_3$ | $NH-CH_2CH_3$ | |
| 1.36. | $OCH_3$ | $OCH_3$ | O | $CH_3$ | $OCH(CH_3)_2$ | |
| 1.37. | $OCH_3$ | $OCH_3$ | O | $CH_3$ | 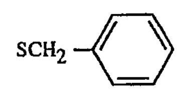 | |
| 1.38. | $OCH_3$ | $OCH_3$ | O | $CH_3$ | 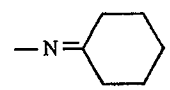 | |
| 1.39. | $OCH_3$ | $OCH_3$ | O | $CH_3$ | 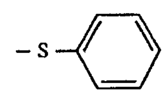 | |

| Nr. | $R_1$ | $R_2$ | X | $R_6$ | $R_7$ | physikal. Daten |
|---|---|---|---|---|---|---|
| 1.40. | $OCH_3$ | $OCH_3$ | O | $CH_3$ | 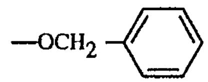 | |
| 1.41. | $OCH_3$ | $OCH_3$ | O | $CH_3$ | 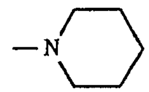 | |
| 1.42. | $OCH_3$ | $OCH_3$ | O | $CH_3$ | $SCF_3$ | |
| 1.43. | $OCH_3$ | $OCH_3$ | O | $CH_3$ | 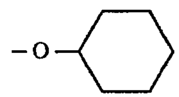 | |
| 1.44. | $OCH_3$ | $OCH_3$ | S | $CH_3$ | 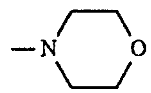 | |
| 1.45. | $OCH_3$ | $OCH_3$ | S | $CH_3$ | $OCH_3$ | |
| 1.46. | $OCH_3$ | $OCH_3$ | O | $CH_2CH_3$ | 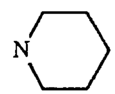 | |
| 1.47. | $OCH_3$ | $OCH_3$ | O | 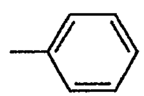 | $OCH_3$ | |

| Nr. | $R_1$ | $R_2$ | X | $R_6$ | $R_7$ | physikal. Daten |
|---|---|---|---|---|---|---|
| 1.48. | $OCH_3$ | $OCH_3$ | O | $CH_3$ | $SCCl_2CHCl_2$ | |
| 1.49. | $OCH_3$ | $OCH_3$ | O | $CH_3$ | $-N=C(CH_3)_2$ | |
| 1.50. | $OCH_3$ | H | O | $CH_3$ | $SCF_3$ | |
| 1.51. | $OCH_3$ | $OCH_3$ | O | $CH_3$ | $-N=CHCH_3$ | |
| 1.52. | $OCH_3$ | $OCH_3$ | O | $CH_3$ | $OCH(CH_3)CH_2CH_3$ | |
| 1.53. | $OCH_3$ | $OCH_3$ | O | 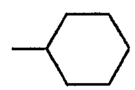 | $-NH_2$ | |
| 1.54. | $OCH_3$ | $OCH_3$ | O | $CH_3$ | $N(CH_3)CH_2CH=CH_2$ | |
| 1.55. | $OCH_3$ | $OCH_3$ | O | $CH_3$ | 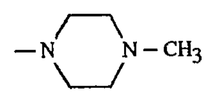 | |
| 1.56. | $OCH_3$ | $OCH_3$ | O | $CH(CH_3)_2$ | $OCH_2CH_3$ | Smp. 116-118°C |
| 1.57. | $-OCH_2O-$ | | O | $CH_3$ | $OCH_3$ | |

| Nr. | $R_1$ | $R_2$ | X | $R_6$ | $R_7$ | physikal. Daten |
|---|---|---|---|---|---|---|
| 1.58. | $-OCH_2O-$ | | O | $CH_3$ | $-N(CH_3)_2$ | |
| 1.59. | $-OCF_2O-$ | | O | $CH_3$ | $OCH_3$ | |
| 1.60. | $-OCH_2CH_2O-$ | | O | $CH_3$ | $OCH_3$ | |
| 1.61. | $-OCH_2O-$ | | O | $CH_3$ | $OCH_2CH_3$ | |
| 1.62. | $-OCH_2O-$ | | O | $CH_2CH_3$ | $OCH_3$ | |
| 1.63. | $-OCF_2O-$ | | O | $CH_3$ | $OC_2H_5$ | |
| 1.64. | $-OCF_2O-$ | | O | $CH_3$ | $-NHCH_3$ | |
| 1.65 | $OCH_3$ | $OCH_3$ | O | $CH_3$ | 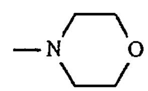 | |
| 1.66. | $OCH_3$ | H | O | $CH_3$ | $-NH-C_2H_5$ | |
| 1.67. | $OCH_3$ | $OCH_3$ | O | $CH_3$ | 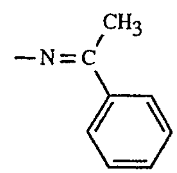 | |
| 1.68. | $OCH_3$ | $OCH_3$ | O | $CH_3$ | $-COCH_3$ | |

| Nr. | $R_1$ | $R_2$ | X | $R_6$ | $R_7$ | physikal. Daten |
|---|---|---|---|---|---|---|
| 1.69. | $OCH_3$ | $OCH_3$ | O | H | 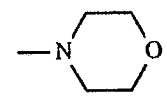 | Smp. 192-193°C |
| 1.70. | $OCH_3$ | $OCH_3$ | O | H | $OCH_2CH_3$ | Smp. 172-173°C |
| 1.71. | $OCH_3$ | $OCH_3$ | O | H | $NHCH_3$ | |
| 1.72. | $OCH_3$ | $OCH_3$ | O | H | $N(CH_3)_2$ | Smp. 190-191°C |
| 1.73. | $OCH_3$ | $OCH_3$ | O | $OCH_3$ | $CH_2$-C≡CH | Smp. 131-132°C |
| 1.74. | $OCH_3$ | $OCH_3$ | O | $OC_2H_5$ | $CH_2$-C≡CH | Smp. 117-119°C |
| 1.75. | $OCH_3$ | $OCH_3$ | O | H | $NH_2$ | Smp. 190-191°C |
| 1.76. | $OCH_3$ | $OCH_3$ | O | H | 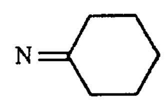 | Smp. 178-179°C |
| 1.77. | $OCH_3$ | $OCH_3$ | O | H | 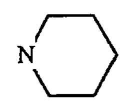 | Smp. 151-152°C |
| 1.78. | $OCH_3$ | $OCH_3$ | O | H | $N=C(CH_3)_2$ | Smp. 200-202°C |

| Nr. | $R_1$ | $R_2$ | X | $R_6$ | $R_7$ | physikal. Daten |
|---|---|---|---|---|---|---|
| 1.79. | $OCH_3$ | $OCH_3$ | O | H | $N=C(CH_3)C_2H_5$ | Smp. 166-168°C |
| 1.80. | $OCH_3$ | $OCH_3$ | O | $CH_3$ | 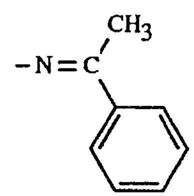 | Smp. 213-214°C |

Tabelle 2:

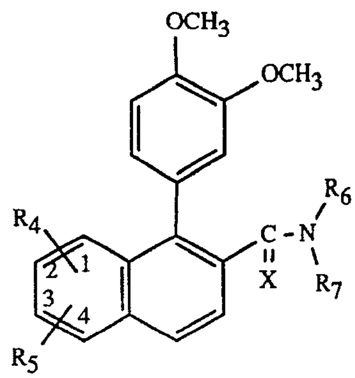

| Nr. | $R_4$ | $R_5$ | X | $R_6$ | $R_7$ | physikal. Daten |
|---|---|---|---|---|---|---|
| 2.1. | 1-$OCH_3$ | H | O | $CH_3$ | $OCH_3$ | |
| 2.2. | 3-Cl | H | O | $CH_3$ | $OCH_3$ | |
| 2.3. | 2-$OCH_3$ | H | O | $CH_3$ | $OCH_3$ | |
| 2.4. | 3-Cl | H | O | $CH_3$ | -NH-$CH_3$ | |
| 2.5. | 2-$OCF_2O$-3 | | O | $CH_3$ | $OCH_3$ | |
| 2.6. | 2-$OCH_2O$-3 | | O | $CH_3$ | $OCH_3$ | |
| 2.7. | 3-$OCH_3$ | H | O | $CH_3$ | $OCH_3$ | |
| 2.8. | 3-Cl | H | O | $C_2H_5$ | $OCH_3$ | |
| 2.9. | 4-$OCH_3$ | H | O | $CH_3$ | $OCH_3$ | |
| 2.10. | 2-Cl | H | O | $CH_3$ | $OCH_3$ | |
| 2.11. | 3-Cl | H | O | $CH_3$ | $SCH_3$ | |
| 2.12. | 3-Cl | H | S | $CH_3$ | $OCH_3$ | |
| 2.13. | 3-Cl | H | O | $CH_3$ | -N=C$(CH_3)_2$ | |
| 2.14. | 3-Cl | H | O | $CH_3$ | $OC_2H_5$ | |
| 2.15. | H | H | O | $CH_3$ | $OCH_2CH=CH_2$ | |

| Nr. | $R_4$ | $R_5$ | X | $R_6$ | $R_7$ | physikal. Daten |
|---|---|---|---|---|---|---|
| 2.16. | H | H | O | $CH_3$ | $OC(CH_3)_3$ | |
| 2.17. | H | H | O | $CH_3$ | 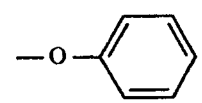 | |
| 2.18. | H | H | O | $CH_3$ | 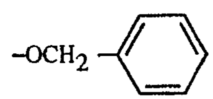 | |

2. Formulierungsbeispiele für Wirkstoffe der Formel I (% = Gewichtsprozent)

| 2.1. Emulsion-Konzentrate | a) | b) | c) |
|---|---|---|---|
| Wirkstoff aus den Tabellen 1 und 2 | 25% | 40% | 50% |
| Ca-Dodecylbenzolsulfonat | 5% | 8% | 6% |
| Rizinusöl-polyäthylenglykoläther (36 Mol Aethylenoxid) | 5% | - | - |
| Tributylphenoyl-polyäthylenglykoläther (30 Mol Aethylenoxid) | - | 12% | 4% |
| Cyclohexanon | - | 15% | 20% |
| Xylolgemisch | 65% | 25% | 20% |

Aus solchen Konzentraten können durch Verdünnen mit Wasser Emulsionen jeder gewünschten Konzentration hergestellt werden.

| 2.2. Lösungen | a) | b) | c) | d) |
|---|---|---|---|---|
| Wirkstoff aus den Tabellen 1 und 2 | 80% | 10% | 5% | 95% |
| Aethylenglykol-monomethyläther | 20% | - | - | - |
| Polyäthylenglykol M G 400 | - | 70% | - | - |
| N-Methyl-2-pyrrolidon | - | 20% | - | - |
| Epoxydiertes Kokosnussöl | - | - | 1% | 5% |
| Benzin (Siedegrenzen 160-190° C) | - | - | 94% | - |
| (MG = Molekulargewicht) | | | | |

Diese Lösungen eignen sich für die Applikation als Mikrodispersionen.

| 2.3. Granulate | a) | b) |
|---|---|---|
| Wirkstoff aus den Tabellen 1 und 2 | 5% | 10% |
| Kaolin | 94% | - |
| Hochdisperse Kieselsäure | 1% | - |
| Attapulgit | - | 90% |

Der Wirkstoff wird in Methylenchlorid gelöst, auf den Träger aufgesprüht und das Lösungsmittel anschliessend im Vakuum abgedampft.

| 2.4. Stäubemittel | a) | b) |
|---|---|---|
| Wirkstoff aus den Tabellen 1 und 2 | 2% | 5% |
| Hochdisperse Kieselsäure | 1% | 5% |
| Talkum | 97% | - |
| Kaolin | - | 90% |

Durch inniges Vermischen auf Trägerstoffe mit dem Wirkstoff erhält man gebrauchsfertige Stäubemittel.

| 2.5. Spritzpulver | a) | b) | c) |
|---|---|---|---|
| Wirkstoff aus den Tabellen 1 und 2 | 25% | 50% | 75% |
| Na-Ligninsulfonat | 5% | 5% | - |
| Na-Laurylsulfat | 3% | - | 5% |
| Na-Diisobutylnaphthalinsulfonat | - | 6% | 10% |
| Octylphenolpolyäthylenglykoläther (7-8 Mol Aethylenoxid) | - | 2% | - |
| Hochdisperse Kieselsäure | 5% | 10% | 10% |
| Kaolin | 62% | 27% | - |

Der Wirkstoff wird mit den Zusatzstoffen gut vermischt und in einer geeigneten Mühle vermahlen. Man erhält Spritzpulver, die sich mit Wasser zu Suspensionen jeder gewünschten Konzentration verdünnen lassen.

3. Biologische Beispiele

Beispiel 3.1: Wirkung geben Plasmopara viticola auf Reben

a) Residual-protektive Wirkung

Im 4-5 Blattstadium werden Rebensämlinge mit einer aus Spritzpulver des Wirkstoffes hergestellten Spritzbrühe (0,02 % Aktivsubstanz) besprüht. Nach 24 Stunden werden die behandelten Pflanzen mit einer Sporangiensuspension des Pilzes infiziert. Nach einer Inkubation während 6 Tagen bei 95-100 % relativer Luftfeuchtigkeit und 20° C wird der Pilzbefall beurteilt.

b) Residual-kurative Wirkung

Im 4-5 Blattstadium werden Rebensämlinge mit einer Sporangiensuspension des Pilzes infiziert. Nach einer Inkubation während 24 Stunden in einer Feuchtkammer bei 95-100 % relativer Luftfeuchtigkeit und 20° C werden die infizierten Pflanzen getrocknet und mit einer aus Spritzpulver des Wirkstoffes hergestellten Spritzbrühe (0,02 % Aktivsubstanz) besprüht. Nach dem Antrocknen des Spritzbelages werden die behandelten Pflanzen wieder in die Feuchtkammer gebracht. Die Beurteilung des Pilzbefalls erfolgt 6 Tage nach der Infektion.

Verbindungen aus den Tabellen zeigen gegen Plasmopara viticola auf Reben eine sehr gute fungizide Wirkung, insbesondere die Wirkstoffe Nr. 1.1, 1.5, 1.6, 1.15, 1.33, 1.44, 1.65 und 2.2 bewirken eine vollständige Unterdrückung des Pilzbefalls (Restbefall 0 bis 5 %). Unbehandelte aber infizierte Kontrollpflanzen weisen dagegen einen Plasmopara-Befall von 100 % auf.

Beispiel 3.2: Wirkung geben Phytophthora auf Tomatenpflanzen

a) Residual-protektive Wirkung

Tomatenpflanzen werden nach 3-wöchiger Anzucht mit einer aus Spritzpulver des Wirkstoffes hergestellten Spritzbrühe (0,02 % Aktivsubstanz) besprüht. Nach 24 Stunden werden die behandelten Pflanzen mit einer Sporangiensuspension des Pilzes infiziert. Die Beurteilung des Pilzbefalls erfolgt nach einer Inkubation der infizierten Pflanzen während 5 Tagen bei 90-100 % relativer Luftfeuchtigkeit und 20° C.

b) Systemische Wirkung

Zu Tomatenpflanzen wird nach dreiwöchiger Anzucht eine aus Spritzpulver des Wirkstoffes hergestellte Spritzbrühe (0,002 % Aktivsubstanz bezogen auf das Erdvolumen) gegeben. Es wird dabei darauf geachtet, dass die Spritzbrühe nicht mit den oberirdischen Pflanzenteilen in Berührung kommt. Nach 48 Stunden werden die behandelten Pflanzen mit einer Sporangiensuspension des Pilzes infiziert. Die Beurteilung des Pilzbefalls erfolgt nach einer Inkubation der infizierten Pflanzen während 5 Tagen bei 90-100 % relativer Luftfeuchtigkeit und 20° C.

Verbindungen aus den Tabellen zeigen eine nachhaltige Wirkung (weniger als 20 % Pilzbefall). Mit den Verbindungen Nr. 1.1, 1.5, 1.6, 1.15, 1.33, 1.44, 1.65 und 2.2 wird ein Befall praktisch vollständig verhindert (0 bis 5 % Befall). Unbehandelte aber infizierte Kontrollpflanzen weisen dagegen einen Phytophthora-Befall von 100 % auf.

## Ansprüche

1. Verbindungen der Formel I

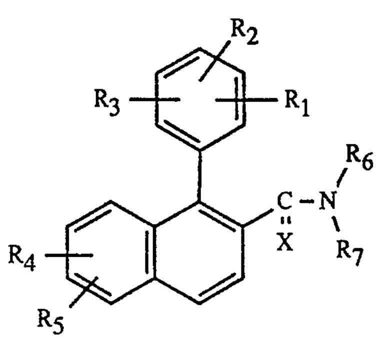

(I)

worin bedeuten:

$R_1$, $R_2$, $R_3$, $R_4$ und $R_5$ unabhängig voneinander Wasserstoff, Halogen, Nitro, Cyano, unsubstituiertes oder durch Halogen und/oder $C_1$-$C_3$-Alkoxy ein- oder mehrfach substituiertes $C_1$-$C_6$-Alkyl, oder $C_3$-$C_6$-Cycloalkyl, $OR_8$, $N(R_9)R_{10}$, $C(O)OR_9$, $CON(R_9)R_{10}$ oder $N(R_9)COR_{11}$, oder worin zwei benachbarte Positionen an den aromatischen Ringen durch eine unsubstituierte oder durch Fluor ein- oder mehrfach substituierte Methylendioxy- oder Aethylendioxy-Gruppe überbrückt sind,

$R_8$ Wasserstoff oder $C_1$-$C_6$Alkyl, das unsubstituiert oder durch $C_1$-$C_3$-Alkoxy oder ein-oder mehrfach durch Halogen substituiert ist, oder $C_3$-$C_4$-Alkenyl, Propargyl, 3-Halogen-2-propinyl,

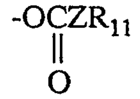 oder $COR_{11}$;

$R_9$ und $R_{10}$ unabhängig voneinander Wasserstoff oder $C_1$-$C_4$-Alkyl;

$R_{11}$ $C_1$-$C_4$-Alkyl;

Z Sauerstoff oder NH;

$R_6$ Wasserstoff, unsubstituiertes oder durch $C_1$-$C_4$-Alkoxy, Halogen und/oder Cyano ein-oder mehrfach substituiertes $C_1$-$C_6$-Alkyl, oder $C_3$-$C_6$-Cycloalkyl, $C_3$-$C_7$-Alkenyl, $C_3$-$C_7$-Alkinyl oder durch Halogen substituiertes $C_3$-$C_7$-Alkinyl oder

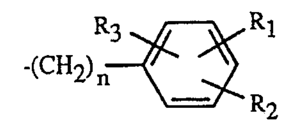 ;

n 0 oder 1;

$R_7$ X-$R_{12}$,

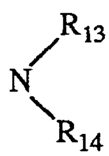 oder 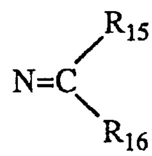 ;

X Sauerstoff oder Schwefel;

$R_{12}$ die Definitionen von $R_6$ oder

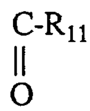 ;

$R_{13}$ und $R_{14}$ unabhängig voneinander die Definitionen von $R_{12}$, oder $R_{13}$ und $R_{14}$ gemeinsam eine $C_4$-$C_7$-Alkylenkette bilden, welche mit dem sie verbindenden Stickstoffatom einen unsubstituierten oder durch $C_1$-$C_4$-Alkyl mono- oder disubstituierten Heterocyclus bilden kann, wobei eine der Methylengruppen aus dieser Kette durch O, S oder $NR_9$ ersetzt sein kann;

$R_{15}$ und $R_{16}$ unabhängig voneinander die Definitionen von $R_6$, oder $R_{15}$ und $R_{16}$ gemeinsam eine $C_4$-$C_7$-Alkylenkette bilden, wobei diese durch $C_1$-$C_4$ Alkyl mono- oder disubstituiert sein kann und/oder eine der Methylengruppe aus dieser Kette durch O, S oder $NR_9$ ersetzt sein kann;

einschliesslich der Säureadditionsalze der Verbindungen der Formel I.

2. Verbindungen der Formel I gemäss Anspruch 1, worin bedeuten:

$R_1$, $R_2$, $R_3$, $R_4$ und $R_5$ unabhängig voneinander Wasserstoff, Halogen, Nitro, Cyano, unsubstituiertes oder durch Halogen und/oder $C_1$-$C_3$-Alkoxy ein- oder mehrfach substituiertes $C_1$-$C_6$-Alkyl, oder $C_3$-$C_6$-Cycloalkyl, $OR_8$ oder $N(R_9)R_{10}$; oder worin zwei benachbarte Positionen an den aromatischen Ringen durch eine unsubstituierte oder durch Fluor ein- oder mehrfach substituierte Methylendioxy- oder Aethylendioxy-Gruppe überbrückt sind;

$R_8$ Wasserstoff oder $C_1$-$C_6$Alkyl, das unsubstituiert oder durch $C_1$-$C_3$-Alkoxy oder ein-oder mehrfach durch Halogen substituiert ist, oder $C_3$-$C_4$-Alkenyl, 2-Propinyl oder 3-Halogen-2-propinyl;
$R_9$ und $R_{10}$ unabhängig voneinander Wasserstoff oder $C_1$-$C_4$-Alkyl;
$R_6$ Wasserstoff, unsubstituiertes oder durch $C_1$-$C_4$-Alkoxy, Halogen und/oder Cyano ein-oder mehrfach substituiertes $C_1$-$C_6$-Alkyl, oder $C_3$-$C_6$-Cycloalkyl, $C_3$-$C_7$-Alkenyl, $C_3$-$C_7$-Alkinyl oder durch Halogen substituiertes $C_3$-$C_7$Alkinyl; $R_7$ X-$R_{12}$,

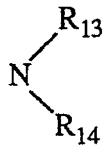 oder 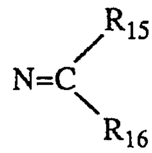 ;

X Sauerstoff oder Schwefel;
$R_{12}$ Wasserstoff, unsubstituiertes oder durch $C_1$-$C_4$-Alkoxy, Halogen und/oder Cyano ein-oder mehrfach substituiertes $C_1$-$C_6$-Alkyl, oder $C_3$-$C_6$-Cycloalkyl, $C_3$-$C_1$-Alkenyl, $C_3$-$C_7$-Alkinyl oder durch Halogen substituiertes $C_3$-$C_7$-Alkinyl oder

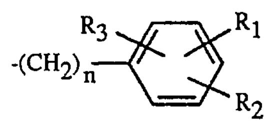 oder 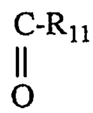 ;

$R_{11}$ $C_1$-$C_4$-Alkyl;
n 0 oder 1;
$R_{13}$ und $R_{14}$ unabhängig voneinander die Definitionen von $R_{12}$, oder $R_{13}$ und $R_{14}$ gemeinsam eine $C_4$-$C_7$-Alkylenkette bilden, welche mit dem sie verbindenden Stickstoffatom einen unsubstituierten oder durch $C_1$-$C_4$-Alkyl mono- oder disubstituierten Heterocyclus bilden kann, wobei eine der Methylengruppen aus dieser Kette durch O, S oder $NR_9$ ersetzt sein kann;
$R_{15}$ und $R_{16}$ unabhängig voneinander Wasserstoff, unsubstituiertes oder durch $C_1$-$C_4$-Alkoxy, Halogen und/oder Cyano ein- oder mehrfach substituiertes $C_1$-$C_6$-Alkyl, oder $C_3$-$C_6$-Cycloalkyl, $C_3$-$C_7$-Alkenyl, $C_3$-$C_7$-Alkinyl oder durch Halogen substituiertes $C_3$-$C_7$-Alkinyl oder

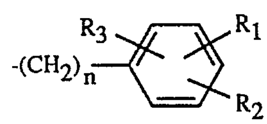 ;

oder $R_{15}$ und $R_{16}$ gemeinsam eine $C_4$-$C_7$-Alkylenkette bilden, wobei diese durch $C_1$-$C_4$ Alkyl mono- oder disubstituiert sein kann und/oder eine der Methylengruppe aus dieser Kette durch O, S oder $NR_9$ ersetzt sein kann; einschliesslich der Säureadditionsalze der Verbindungen der Formel I.

3. Verbindungen der Formel I gemäss Anspruch 1, worin bedeuten:
$R_1$, $R_2$, $R_3$, $R_4$ und $R_5$ unabhängig voneinander Wasserstoff, Halogen, Nitro, Cyano, unsubstituiertes oder durch Halogen und/oder $C_1$-$C_3$-Alkoxy ein- oder mehrfach substituiertes $C_1$-$C_6$-Alkyl, oder $C_3$-$C_6$-Cycloalkyl, $OR_8$ oder $N(R_9)R_{10}$; oder worin zwei benachbarte Positionen an den aromatischen Ringen durch eine unsubstituierte oder durch Fluor ein- oder zweifach substituierte Methylendioxy-Gruppe überbrückt sind,
$R_8$ Wasserstoff oder $C_1$-$C_6$Alkyl, das unsubstituiert oder durch $C_1$-$C_3$-Alkoxy oder ein-oder mehrfach durch Halogen substituiert ist, oder $C_3$-$C_4$-Alkenyl, Propargyl oder 3-Halogen-2-propinyl;
$R_9$ und $R_{10}$ unabhängig voneinander Wasserstoff oder $C_1$-$C_4$-Alkyl;
$R_6$ Wasserstoff, unsubstituiertes oder durch $C_1$-$C_2$-Alkoxy, Halogen und/oder Cyano ein-oder mehrfach substituiertes $C_1$-$C_4$-Alkyl, oder $C_3$-$C_6$-Cycloalkyl, $C_3$-$C_5$-Alkenyl, $C_3$-$C_5$- Alkinyl oder 3-Halogen-2-propinyl;
$R_7$ X-$R_{12}$,

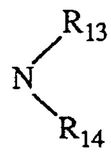 oder 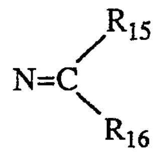 ;

X Sauerstoff oder Schwefel;

$R_{12}$ Wasserstoff, unsubstituiertes oder durch $C_1$-$C_4$-Alkoxy, Halogen und/oder Cyano ein-oder mehrfach substituiertes $C_1$-$C_6$-Alkyl, oder $C_3$-$C_6$-Cycloalkyl, $C_3$-$C_7$-Alkenyl, $C_3$-$C_7$-Alkinyl, 3-Halogen-2-propinyl oder

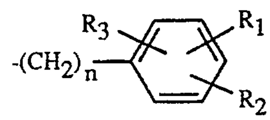 ;

n 0 oder 1;

$R_{13}$ und $R_{14}$ unabhängig voneinander die Definitionen von $R_{12}$, oder $R_{13}$ und $R_{14}$ gemeinsam eine $C_4$-$C_7$-Alkylenkette bilden, welche mit dem sie verbindenden Stickstoffatom einen unsubstituierten oder durch $C_1$-$C_4$-Alkyl mono- oder disubstituierten Heterocyclus bilden kann, wobei eine der Methylengruppen aus dieser Kette durch O oder S ersetzt sein kann;

$R_{15}$ und $R_{16}$ unabhängig voneinander Wasserstoff, unsubstituiertes oder durch $C_1$-$C_4$-Alkoxy, Halogen und/oder Cyano ein- oder mehrfach substituiertes $C_1$-$C_6$-Alkyl, oder $C_3$-$C_6$-Cycloalkyl, $C_3$-$C_7$-Alkenyl, $C_3$-$C_7$-Alkinyl, 3-Halogen-2-propinyl oder

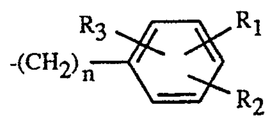 ;

oder $R_{15}$ und $R_{16}$ gemeinsam eine $C_4$-$C_7$-Alkylenkette bilden, wobei diese durch $C_1$-$C_4$-Alkyl mono- oder disubstituiert sein kann und/oder eine der Methylengruppe aus dieser Kette durch O oder S ersetzt sein kann;

einschliesslich der Säureadditionsalze der Verbindungen der Formel I.

4. Verbindungen der Formel I gemäss Anspruch 1, worin bedeuten:

$R_1$, $R_2$, $R_3$, $R_4$ und $R_5$ unabhängig voneinander Wasserstoff, Halogen, Nitro, Cyano, unsubstituiertes oder durch Halogen und/oder $C_1$-$C_3$-Alkoxy ein- oder mehrfach substituiertes $C_1$-$C_6$-Alkyl, oder $C_3$-$C_6$-Cycloalkyl, $OR_8$ oder $N(R_9)R_{10}$; oder worin zwei benachbarte Positionen an den aromatischen Ringen durch eine unsubstituierte oder durch Fluor ein- oder zweifach substituierte Methylendioxy-Gruppe überbrückt sind,

$R_8$ Wasserstoff oder $C_1$-$C_6$Alkyl, das unsubstituiert oder durch $C_1$-$C_3$-Alkoxy oder ein-oder mehrfach durch Halogen substituiert ist, oder $C_3$-$C_4$-Alkenyl, Propargyl oder 3-Halogen-2-propinyl;

$R_9$ und $R_{10}$ unabhängig voneinander Wasserstoff oder $C_1$-$C_4$-Alkyl;

$R_6$ Wasserstoff, unsubstituiertes oder durch $C_1$-$C_2$-Alkoxy, Halogen und/oder Cyano ein-oder mehrfach substituiertes $C_1$-$C_4$-Alkyl, oder $C_3$-$C_6$-Cycloalkyl, $C_3$-$C_5$-Alkenyl, $C_3$-$C_5$-Alkinyl oder 3-Halogen-2-propinyl;

$R_7$ X-$R_{12}$,

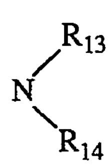 oder 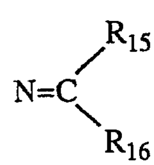 ;

X Sauerstoff oder Schwefel;

$R_{12}$ Wasserstoff, unsubstituiertes oder durch $C_1$-$C_2$-Alkoxy, Halogen und/oder Cyano ein-oder mehrfach substituiertes $C_1$-$C_4$-Alkyl, oder $C_3$-$C_6$-Cycloalkyl, $C_3$-$C_5$-Alkenyl, $C_3$-$C_5$-Alkinyl oder 3-Halogen-2-propinyl;

$R_{13}$ und $R_{14}$ unabhängig voneinander die Definitionen von $R_{12}$, oder $R_{13}$ und $R_{14}$ gemeinsam eine $C_4$-$C_7$-Alkylenkette bilden, welche mit dem sie verbindenden Stickstoffatom einen unsubstituierten oder durch $C_1$-$C_4$-Alkyl mono- oder disubstituierten Heterocyclus bilden kann, wobei eine der Methylengruppen aus dieser Kette durch O oder S ersetzt sein kann;

$R_{15}$ und $R_{16}$ unabhängig voneinander Wasserstoff, unsubstituiertes oder durch $C_1$-$C_2$-Alkoxy, Halogen und/oder Cyano ein- oder mehrfach substituiertes $C_1$-$C_4$-Alkyl, oder $C_3$-$C_6$-Cycloalkyl, $C_3$-$C_5$-Alkenyl, $C_3$-$C_5$-Alkinyl oder 3-Halogen-2-propinyl; oder $R_{15}$ und $R_{16}$ gemeinsam eine $C_4$-$C_7$-Alkylenkette bilden, wobei diese durch $C_1$-$C_4$-Alkyl mono- oder disubstituiert sein kann und/oder eine der Methylengruppe aus dieser Kette durch O oder S ersetzt sein kann;

einschliesslich der Säureadditionssalze der Verbindungen der Formel I.

5. Verbindungen der Formel I gemäss Anspruch 1, worin bedeuten:

$R_1$, $R_2$ und $R_3$ unabhängig voneinander Wasserstoff, Halogen, unsubstituiertes oder durch Halogen und/oder $C_1$-$C_2$-Alkoxy ein- oder mehrfach substituiertes $C_1$-$C_3$-Alkyl, oder $OR_8$, $N(R_9)R_{10}$ oder worin zwei benachbarte Positionen an den aromatischen Ringen durch Difluormethylendioxy überbrückt sind;

$R_8$ Wasserstoff oder $C_1$-$C_3$ Alkyl, das unsubstituiert oder durch $C_1$-$C_3$-Alkoxy oder ein-oder mehrfach durch Halogen substituiert ist;

$R_4$ und $R_5$ unabhängig voneinander Wasserstoff, Halogen, $NO_2$ oder $C_1$-$C_3$-Alkoxy, oder worin zwei benachbarte Positionen an den aromatischen Ringen durch eine unsubstituierte oder durch Fluor ein- oder zweifach substituierte Methylendioxy-Gruppe überbrückt sind;

$R_9$ und $R_{10}$ unabhängig voneinander Wasserstoff oder $C_1$-$C_4$-Alkyl;

$R_6$ Wasserstoff, unsubstituiertes oder durch $C_1$-$C_2$-Alkoxy, Halogen und/oder Cyano substituiertes $C_1$-$C_4$-Alkyl, oder 2-Allyl, 2-Propinyl oder 3-Jod-2-propinyl;

$R_7$ X-$R_{12}$,

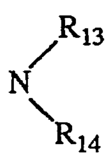 oder 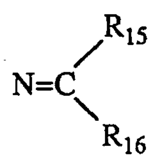 ;

X Sauerstoff oder Schwefel;

$R_{12}$ Wasserstoff, $C_1$-$C_4$-Alkyl, 2-Allyl oder 2-Propinyl;

$R_{13}$ und $R_{14}$ unabhängig voneinander die Definitionen von $R_{12}$, oder $R_{13}$ und $R_{14}$ gemeinsam eine $C_4$-$C_7$-Alkylenkette bilden, welche mit dem sie verbindenden Stickstoffatom einen unsubstituierten Heterocyclus bilden kann, wobei eine der Methylengruppen aus dieser Kette durch O oder S ersetzt sein kann;

$R_{15}$ und $R_{16}$ unabhängig voneinander die Definitionen von $R_{13}$ oder $R_{14}$.

6. Verbindungen der Formel I gemäss Anspruch 1, worin bedeuten:

$R_1$, $R_2$ und $R_3$ unabhängig voneinander Wasserstoff, Halogen, $CF_3$, $OR_8$ oder $N(R_9)R_{10}$;

$R_8$ Wasserstoff, $C_1$-$C_3$-Alkyl oder $CHF_2$;

$R_9$ und $R_{10}$ unabhängig voneinander Wasserstoff oder $C_1$-$C_3$-Alkyl;

$R_4$ und $R_5$ unabhängig voneinander Wasserstoff, Halogen oder Methoxy;

$R_6$ Wasserstoff, $C_1$-$C_4$-Alkyl oder $C_1$-$C_3$-Cyanoalkyl;

$R_7$ X-$R_{12}$,

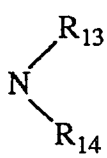 oder 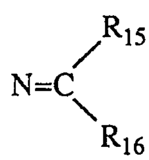 ;

X Sauerstoff oder Schwefel;

$R_{12}$ Wasserstoff oder $C_1$-$C_4$-Alkyl;

$R_{13}$, $R_{14}$, $R_{15}$ und $R_{16}$ unabhängig voneinander die Definitionen von $R_{12}$, oder $R_{13}$ und $R_{14}$ und/oder $R_{15}$ und $R_{16}$ gemeinsam eine $C_4$-$C_5$-Alkylenkette bilden, wobei eine der Methylengruppe durch O oder S ersetzt sein kann.

7. 1-(3,4-Dimethoxyphenyl)-2-naphthoesäure-N-methyl-N-methoxy-amid;

1-(3,4-Dimethoxyphenyl)-2-naphthoesäure-N-methyl-N-ethoxy-amid;
1-(3,4-Dimethoxyphenyl)-2-naphthoesäure-N-ethyl-N-methoxy-amid;
1-(3,4-Dimethoxyphenyl)-2-naphthoesäure-N-ethyl-N-ethoxy-amid;
1,2-Dimethyl-1-[1-(3,4-dimethoxyphenyl)-2-naphthoyl]-hydrazin;
1,2,2-Trimethyl-1-[1-(3,4-dimethoxyphenyl)-2-naphthoyl]-hydrazin;
1-Methyl-1-[1-(3,4-dimethoxyphenyl)-2-naphthoyl]-hydrazono-isopropan.

8. Verfahren zur Herstellung der Verbindungen der Formel I in Anspruch 1, gekennzeichnet durch

A) Umsetzung einer Aminoverbindung der Formel II

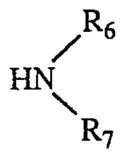

(II)

mit einem Naphthoesäurederivat der Formel III

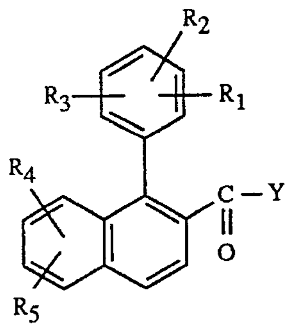

(III),

worin Y OH, Halogen oder $C_1$-$C_4$-Alkoxy bedeutet, zu einer Verbindung der Formel Ia

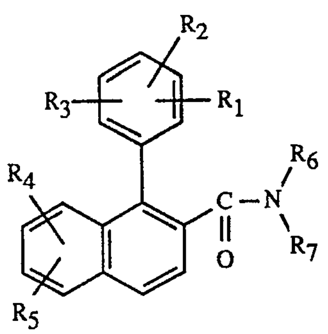

(Ia),

worin $R_1$-$R_7$ die unter Formel I angegebenen Bedeutungen besitzen, bei Temperaturen von -25° bis 150° C, vorzugsweise von -10° C bis zur Siedetemperatur des Reaktionsgemisches, in Gegenwart eines säureaktivierenden und/oder eines wasserentziehenden Mittels in einem reaktionsinerten Lösungsmittel oder Lösungsmittelgemisch;

B) Umsetzung einer Verbindung der Formel Ia mit Phosphorpentasulfid zu einer Verbindung der Formel Ib

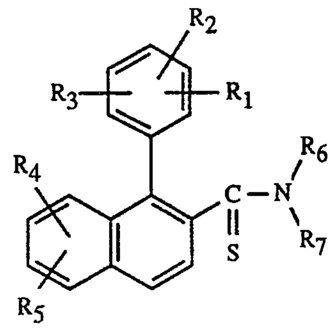

(Ib),

worin $R_1$-$R_7$ die unter Formel I angegebenen Bedeutungen besitzen, bei Temperaturen von 0° bis 120° C, in inerten Lösungsmitteln;

C) Umsetzung einer Verbindung der Formel Ic

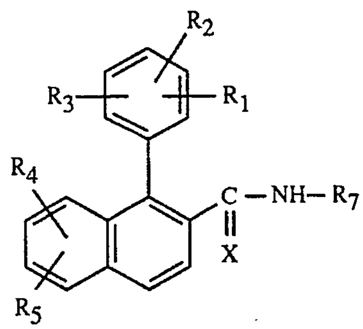

(Ic),

worin $R_1$-$R_7$ und X die unter Formel I angegebenen Bedeutungen darstellen, mit einer Verbindung der Formel IV

$Q-R_6$ (IV),

worin Q eine nucleophile Abgangsgruppe darstellt und die Bedeutung von $R_6$ unter Formel I angegeben ist, bei Temperaturen von 0° bis 220° C in reaktionsinternen Lösungsmitteln;

D) Umsetzung eines Ketons der Formel V

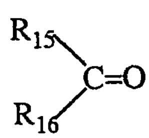

(V)

mit einer Hydrazinverbindung der Formel Ie

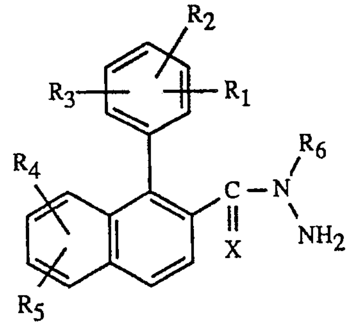

(Ie)

oder einem Salz davon zu einer Verbindung der Formel Id

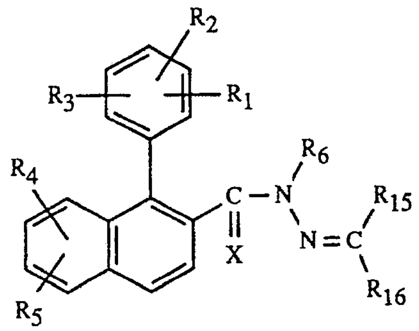

(Id),

worin $R_1$-$R_{16}$ und X die unter Formel I angegebenen Bedeutungen besitzen, bei Temperaturen von -20° bis 200° C in einem reaktionsinerten Lösungsmittel oder in der Schmelze der Reaktionsteilnehmer sowie in Gegenwart eines Katalysators oder ohne Katalysator;

E) Umsetzung einer Verbindung der Formel If

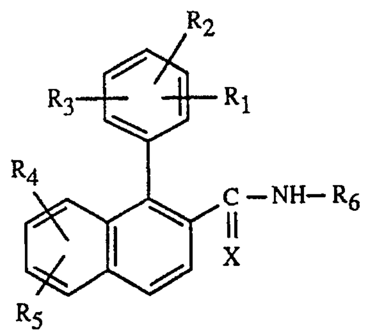

(If)

mit einer sSchwefelverbindung der Formel VI

Hal-S-$R_{12}$ (VI)

zu einer Verbindung der Formel Ig

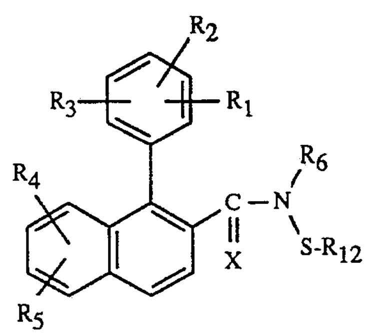

(Ig),

worin $R_1$-$R_{12}$ und X die unter Formel I angegebenen Bedeutungen besitzen, bei Temperaturen von -30° bis 150° C in reaktionsinerten Lösungsmitteln oder Gemischen davon in Gegenwart eines säurebindenden Mittels.

9. Mittel zum Schutz von Pflanzen gegen den Befall durch Mikroorganismen, dadurch gekennzeichnet, dass es neben üblichen Träger- und Hilfsstoffen als aktive Komponente mindestens eine Verbindung gemäss Anspruch 1 enthält.

10. Mittel gemäss Anspruch 9, dadurch gekennzeichnet, dass es als aktive Komponente mindestens eine Verbindung gemäss den Ansprüchen 2 bis 6 enthält.

11. Mittel gemäss Anspruch 9, dadurch gekennzeichnet, dass es als aktive Komponente eine Verbindung der Formel I gemäss Anspruch 7 enthält.

12. Verfahren zur Herstellung eines agrochemischen Mittels von Anspruch 9, dadurch gekennzeichnet, dass man mindestens eine gemäss Anspruch 1 definierte Verbindung mit geeigneten festen oder flüssigen Träger- und Hilfsstoffen innig vermischt.

13. Verwendung von Verbindungen gemäss Anspruch 1 zum Schutz von Pflanzen gegen den Befall durch phytopathogene Mikroorganismen.

14. Verwendung von Verbindungen gemäss einem der Ansprüche 2 bis 7 zum Schutz von Pflanzen gegen den Befall durch phytopathogene Mikroorganismen.

15. Verfahren zum Schutz von Pflanzen gegen den Befall durch phytopathogene Mikroorganismen, dadurch gekennzeichnet, dass man als Wirkstoff eine Verbindung gemäss Anspruch 1 auf die Pflanze oder deren Standort appliziert.

16. Verfahren zum Schutz von Pflanzen gegen den Befall durch phytopathogene Mikroorganismen, dadurch gekennzeichnet, dass man als Wirkstoff eine Verbindung gemäss einem der Ansprüche 2 bis 7 auf die Pflanze oder deren Standort appliziert.

17. Verfahren gemäss den Ansprüchen 15 und 16, dadurch gekennzeichnet, dass es sich bei den phytopathogenen Mikroorganismen um Pilzorganismen handelt.